# EUROPEAN PATENT APPLICATION

(11) **EP 4 176 899 A1**
(43) Date of publication of application: **10.05.2023**
(21) Application number: 22202459.8
(22) Date of filing: 20.08.2014
(51) Int. Cl.: A61K 39/12, A61K 39/00

(54) **PORCINE CIRCOVIRUS TYPE 2 (PCV2) SUBUNIT VACCINE**

(30) Priority: 23.08.2013 US 201361869353 P
(62) Divisional of application: 14761742.7
(71) Applicant: Boehringer Ingelheim Animal Health USA Inc., Duluth, GA 30096 (US)
(72) Inventor: Haiwick, Gregory, Ridgefield, Connecticut 06877-0368 (US); Eichmeyer, Marc Allan, Ridgefield, Connecticut 06877-0368 (US); Hernandez, Luis Alejandro, Ridgefield, Connecticut 06877-0368 (US); Johnson, Wesley Scott, Ridgefield, Connecticut 06877-0368 (US); Muehlenthaler, Christine Margaret, Ridgefield, Connecticut 06877-0368 (US); Vaughn, Eric Martin, Ridgefield, Connecticut 06877-0368 (US)
(74) Representative: Hoffmann Eitle

(57) **Abstract**

Vaccination methods to control PCV2 infection with different PCV2 subtypes are disclosed. Specifically, a PCV2 subtype b (PCV2b) ORF2 proteins or immunogenic compositions comprising a PCV2b ORF2 protein are used in a method for the treatment or prevention of an infection with PCV2 of a different subtype, the reduction, prevention or treatment of clinical signs caused by an infection with PCV2 of a different subtype, or the prevention or treatment of a disease caused by an infection with PCV2 of a different subtype.

## Description

### SEQUENCE LISTING

This application contains a sequence listing in accordance with 37 C.F.R. 1.821 - 1.825. The sequence listing accompanying this application is hereby incorporated by reference in its entirety.

### BACKGROUND OF THE INVENTION

Porcine circovirus type 2 (PCV2) is a small (17 -22 nm in diameter), icosahedral, non-enveloped DNA virus, which contains a single-stranded circular genome. PCV2 shares approximately 80% sequence identity with porcine circovirus type 1 (PCV-1). However, in contrast with PCV1, which is generally non-virulent, swine infected with PCV2 exhibit a syndrome commonly referred to as Post-weaning Multisystemic Wasting Syndrome (PMWS). PMWS is clinically characterized by wasting, paleness of the skin, unthriftiness, respiratory distress, diarrhea, icterus, and jaundice. In some affected swine, a combination of all signs will be apparent while other swine will only have one or two of these clinical signs. During necropsy, microscopic and macroscopic lesions also appear on multiple tissues and organs, with lymphoid organs being the most common site for lesions. A strong correlation has been observed between the amount of PCV2 nucleic acid or antigen and the severity of microscopic lymphoid lesions. Mortality rates for swine infected with PCV2 can approach 80%. In addition to PMWS, PCV2 has been associated with several other infections including pseudorabies, porcine reproductive and respiratory syndrome (PRRS), Glasser's disease, streptococcal meningitis, salmonellosis, postweaning colibacillosis, dietetic hepatosis, and suppurative bronchopneumonia.

Currently, there are three subtypes of PCV2 known (PCV2a, PCV2b and PCV2c), which are classified according to a unified nomenclature for PCV2 genotypes (Segales, J. et al., 2008, PCV-2 genotype definition and nomenclature, Vet Rec 162:867-8). Two further subtypes (PCV2d and PCV2e) have been proposed (Wang et al. Virus Res. 2009 145(1):151-6) but, however, it was demonstrated later that they belong to the PCV2a and PCV2b clusters (Cortey et al. Vet Microbiol. 2011 149(3-4):522-32011). According to this unified nomenclature for PCV2 genotypes the orf2 gene is used to perform genotyping for pcv-2, wherein the geotyping is based on the proportion of nucleotide sites at which two sequences being compared are different (p distance).This value is obtained by dividing the number of nucleotide differences by the total number of nucleotides compared (Kumar et al. 2001 Bioinformatics 17, 1244-1245) and subsequently, the construction of a p distance/frequency histogram enables to determine potential cut-off values to distinguish different genotypes (Rogers and Harpending 1992 Molecular Biology and Evolution 9, 552-569 ; Biagini et al. 1999 Journal of General Virology 80, 419-424). Using this methodology, orf2 pcv-2 sequences are assigned to different genotypes when the genetic distance between them is 0.035.

US 2011/0305725 A1 describes a study to test a new vaccine formulation in pigs to assess its efficacy against porcine circovirus and *M. hyopneumoniae.* During the course of this study, it was observed that several of the pigs in the control and vaccinated groups exhibited clinical signs of PMWS. It was then confirmed that these pigs were exposed to environmental PCV2 prior to challenge. Molecular analysis on blood and tissue samples from these pigs revealed that they harbored a type 2B strain that was different than the strain used for challenge (paragraph [0152] of US 2011/0305725 A1).

Thus, there is a need of new and enhanced vaccination approaches to control PCV2 infection with different PCV2 subtypes.

### SUMMARY OF THE INVENTION

The solution to the above technical problem is achieved by the description and the embodiments characterized in the claims.

Thus, the invention in its different aspects is implemented according to the claims.

The invention is based on the surprising finding that the administration of only one dose of PCV2 subtype b (PCV2b) ORF2 protein, in particular if it is contained in an immunogenic composition, is sufficient for reducing and preventing clinical signs caused by an infection with PCV2a or PCV2b.

### BRIEF DESCRIPTION OF THE DRAWINGS

Figure 1 demonstrates Lymphoid Depletion Results 28 Days Post Challenge. Percentage of pig's positive exhibiting lymphoid depletion from tonsil, iliac, tracheobronchial, and mesenteric lymph nodes. Pigs vaccinated and challenged with PCV2a and pigs vaccinated and challenged with PCV2b shown.
Figure 2 demonstrates Lymphoid Inflammation Results 28 Days Post Challenge. Percentage of pig's positive exhibiting lymphoid inflammation from tonsil, iliac, tracheobronchial, and mesenteric lymph nodes. Pigs vaccinated and challenged with PCV2a and pigs vaccinated and challenged with PCV2b shown.
Figure 3 demonstrates IHC Tissue Results 28 Days Post Challenge. Percentage of pig's positive IHC positive from the following tissues; tonsil, iliac, tracheobronchial, and mesenteric lymph nodes. Pigs vaccinated and challenged with PCV2a and pigs vaccinated and challenged with PCV2b shown.
Figure 4 demonstrates Average Lung Inflammation Score 28 Days Post Challenge. The average group Lung Inflammation score of pigs 28 days post challenge. Pigs vaccinated and challenged with PCV2a and pigs vaccinated and challenged with PCV2b shown.
Figure 5 demonstrates PCV2 Viremia by Treatment Group 28 Days Post Challenge. Percentage of pigs positive by PCR using PCV2a/2b primers and PCV2b specific primers. Pigs vaccinated and challenged with PCV2a and pigs vaccinated and challenged with PCV2b shown.
Figure 6 demonstrates the total severity score, all lymphoidal tissue, which was used for determining the mitigated fraction for depletion, inflammation and colonization per group.
Figure 7 demonstrates the PCV2b viremia after challenge, wherein reduction in viremia was observed after day 28.

### DETAILED DESCRIPTION OF THE PREFERRED EMBODIMENT

The invention is thus in particular based on PCV2b ORF2 protein or on an immunogenic composition comprising PCV2b ORF2 protein, respectively, wherein said PCV2b ORF2 protein or said immunogenic composition comprising PCV2b ORF2 protein may be used for particular purposes.

In one aspect, the invention thus relates to PCV2 subtype b (PCV2b) ORF2 protein or an immunogenic composition comprising PCV2b ORF2 protein for use in a method for the treatment or prevention of an infection with PCV2, the reduction, prevention or treatment of clinical signs caused by an infection with PCV2, or the prevention or treatment of a disease caused by an infection with PCV2.

The invention also provides a method for the treatment or prevention of an infection with PCV2, the reduction, prevention or treatment of clinical signs caused by an infection with PCV2, or the prevention or treatment of a disease caused by an infection with PCV2, comprising administering PCV2b ORF2 protein or an immunogenic composition comprising PCV2b ORF2 protein to an animal, in particular to an animal in need thereof.

Also, the invention provides the use of PCV2b ORF2 protein or of an immunogenic composition comprising PCV2b ORF2 protein for the preparation of a medicament for the treatment or prevention of an infection with PCV2, the reduction, prevention or treatment of clinical signs caused by an infection with PCV2, or the treatment or prevention of a disease caused by an infection with PCV2.

Particularly, in the context of the present invention, a PCV2b ORF2 protein or immunogenic composition comprising PCV2b ORF2 protein is preferred, wherein said PCV2b ORF2 protein is a recombinant PCV2b ORF2 protein, more preferably a recombinant baculovirus expressed PCV2b ORF2 protein.

In particular, for the purpose of the present invention, a PCV2b ORF2 protein or immunogenic composition comprising PCV2b ORF2 protein is preferred, wherein said PCV2b ORF2 protein comprises of consists of a sequence that is at least 94%, preferably at least 95%, more preferably at least 96%, still more preferably at least 97%, yet more preferably at least 98%, and most preferably at least 99% identical to the sequence of SEQ ID No: 1.

The terms "recombinant protein" refers to a polypeptide which is produced by recombinant DNA techniques. An example of such techniques includes the case when DNA encoding the expressed protein is inserted into a suitable expression vector, preferably a baculovirus expression vector, which is in turn used to transform a host cell to produce the protein or polypeptide encoded by the DNA.

The term "recombinant protein", as used herein, thus in particular refers to a protein molecule which is expressed from a recombinant DNA molecule.

It is further understood that the term "recombinant protein consisting of a sequence" in particular also concerns any cotranslational and/or posttranslational modification or modifications of the sequence affected by the cell in which the polypeptide is expressed. Thus, the term "recombinant polypedtide consisting of a sequence", as described herein, is also directed to the sequence having one or more modifications effected by the cell in which the polypeptide is expressed, in particular modifications of amino acid residues effected in the protein biosynthesis and/or protein processing, preferably selected from the group consisting of glycosylations, phosphorylations, and acetylations.

In a preferred aspect, the infection with PCV2, as described herein, is an infection with PCV2 subtype b (PCV2b) and/or an infection with PCV2 of a subtype other than subtype 2b.

As used herein, the term "infection with PCV2" is equivalent to the term "PCV2 infection".

In particular, the infection with PCV2 of a subtype other than subtype 2b, as mentioned herein, is an infection with PCV2 subtype a (PCV2a) and/or PCV2 subtype c (PCV2c), and is preferably an infection with PCV2a.

The term "PCV2 subtype b (PCV2b) ORF2 protein", as described herein, relates to the protein encoded by the ORF2 gene of a PCV-2b as defined by the standardized nomenclature for PCV2 genotype definition (Segales, J. et al., 2008, PCV-2 genotype definition and nomenclature, Vet Rec 162:867-8) which is incorporated herein by reference).

According to another preferred aspect, the infection with PCV2 of a subtype other than subtype 2b, as described herein, is a concurrent infection with (i) PCV2 of a subtype other than subtype 2b and (ii) PCV2b, in particular a concurrent infection with PCV2a and PCV2b.

The terms "PCV2a", "PCV2b" and "PCV2c", respectively, as described herein, relate to PCV-2a, PCV-2b and PCV-2c, respectively, according to the standardized nomenclature for PCV2 genotype definition (Segales, J. et al., 2008, PCV-2 genotype definition and nomenclature, Vet Rec 162:867-8, which is incorporated herein by reference).

In particular, the infection with PCV2b, as mentioned herein, is an infection with (i) a PCV2 comprising a polypeptide that is at least 94%, preferably at least 95%, more preferably at least 96%, still more preferably at least 97%, yet more preferably at least 98%, and most preferably at least 99% identical to the sequence of SEQ ID NO:1 or (***ii***) a PCV2 comprising a polynucleotide which comprises a sequence encoding a polypeptide that is at least 94%, preferably at least 95%, more preferably at least 96%, still more preferably at least 97%, yet more preferably at least 98%, and most preferably at least 99% identical to the sequence of SEQ ID NO:1.

As used herein, it is in particular understood that the term "identical to the sequence of SEQ ID NO: X" is equivalent to the term "identical to the sequence of SEQ ID NO: X over the length of SEQ ID NO: X" or to the term "identical to the sequence of SEQ ID NO: X over the whole length of SEQ ID NO: X", respectively. In this context, "X" is any integer selected from 1 to 2 so that "SEQ ID NO: X" represents any of the SEQ ID NOs mentioned herein.

Preferably, the infection with PCV2a, as described herein, is an infection with (*i*) a PCV2 comprising a polypeptide that is at least 94%, preferably at least 95%, more preferably at least 96%, still more preferably at least 97%, yet more preferably at least 98%, and most preferably at least 99% identical to the sequence of SEQ ID NO:2 or (*ii*) a PCV2 comprising a polynucleotide which comprises a sequence encoding a polypeptide that is at least 94 %, preferably at least 95%, more preferably at least 96%, still more preferably at least 97%, yet more preferably at least 98%, and most preferably at least 99% identical to the sequence of SEQ ID NO:2.

Preferably, in the context of the present invention, the treatment or prevention of an infection with PCV2 is based on or comprises or consists of the induction of an immune response against said PCV2, the clinical signs, as mentioned herein, are selected from the group consisting of lymphoid depletion, lymphoid inflammation, positive IHC for PCV2 antigen of lymphoid tissue, viremia, nasal shedding, pyrexia, reduced average daily weight gain, lung inflammation, positive IHC for PCV2 antigen of lung tissue, increased mortality, and/or the disease, as mentioned herein, is PMWS.

In particular, in the context of the present invention, the treatment or prevention of an infection with PCV2 of a subtype other than 2b is based on or comprises or consists of the induction of an immune response against said PCV2 of a subtype other than 2b or the concurrent induction of an immune response against said PCV2 of a subtype other than 2b and PCV2b.

The term "prevention" or "reduction" or "preventing" or "reducing", respectively, as used herein, means, but is not limited to a process which includes the administration of a PCV2 antigen, namely of the PCV2b ORF2 protein according to the invention, which is included in the composition of the invention, to an animal, wherein said PCV2 antigen, when administered to said animal elicits or is able to elicit an immune response in said animal against PCV2. Altogether, such treatment results in reduction of the clinical signs of a disease caused by PCV2 or of clinical signs associated with PCV2 infection, respectively. More specifically, the term "prevention" or "preventing", as used herein, means generally a process of prophylaxis in which an animal is exposed to the immunogenic composition of the present invention prior to the induction or onset of the disease process caused by PCV2.

Herein "reduction of clinical signs associated with PCV2 infection" means, but is not limited to, reducing the number of infected subjects in a group, reducing or eliminating the number of subjects exhibiting clinical signs of infection, or reducing the severity of any clinical signs that are present in the subjects, in comparison to wild-type infection. For example, it should refer to any reduction of pathogen load, pathogen shedding, reduction in pathogen transmission, or reduction of any clinical sign symptomatic of PCV2 infection. Preferably these clinical signs are reduced in subjects receiving the composition of the present invention by at least 10% in comparison to subjects not receiving the composition and may become infected. More preferably, clinical signs are reduced in subjects receiving the composition of the present invention by at least 20%, preferably by at least 30%, more preferably by at least 40%, and even more preferably by at least 50%.

The term "reduction of viremia" means, but is not limited to, the reduction of PCV2 virus entering the bloodstream of an animal, wherein the viremia level, i.e. the number of PCV2 RNA copies per mL of blood serum or the number of plaque forming colonies per deciliter of blood serum, is reduced in the blood serum of subjects receiving the composition of the present invention by at least 50% in comparison to subjects not receiving the composition and may become infected. More preferably, the viremia level is reduced in subjects receiving the composition of the present invention by at least 90%, preferably by at least 99.9%, more preferably by at least 99.99%, and even more preferably by at least 99.999%.

As used herein, the term "viremia" is particularly understood as a condition in which PCV2 particles reproduce and circulate in the bloodstream of an animal.

The term "animal" as used herein, in particular relates to a mammal, preferably to swine, more preferably to a pig, most preferably to a piglet.

According to a particular preferred aspect of the invention, the PCV2b ORF2 protein or the immunogenic composition is administered only once.

Preferably, in the context of the present invention, the PCV2b ORF2 protein or the immunogenic composition is to be administered or is administered, respectively, in particular only once, to an animal, preferably to a swine, more preferably to a pig, in particular preferably to a piglet.

The present invention overcomes the problems inherent in the prior art and provides a distinct advance in the state of the art. According to another aspect, the present invention also provides a method for the treatment or prevention of a PCV2 infection or for reduction of clinical signs caused by or associated with a PCV2 infection in animals, preferably animals having anti-PCV2 antibodies, comprising the step of administering an effective amount of PCV2b ORF2 protein or an immunogenic composition comprising PCV2b ORF2 protein to that animal in need of such treatment.

The terms "vaccine" or "immunogenic composition" (both terms are used synonymously) as used herein refers to any pharmaceutical composition containing a PCV2b ORF2 protein, which composition can be used to prevent or treat a PCV2 infection-associated disease or condition in a subject. A preferred immunogenic composition can induce, stimulate or enhance the immune response against PCV2. The term thus encompasses both subunit immunogenic compositions, as described below, as well as compositions containing whole killed, or attenuated and/or inactivated PCV2b.

According to another aspect, the present invention also provides a method for the treatment or prevention of a PCV2 infection or for reduction of clinical signs caused by or associated with a PCV2 infection in animals, preferably animals having anti-PCV2 antibodies, in particular maternal derived anti-PCV2 antibodies, comprising the step of administering an effective amount of PCV2b ORF2 protein or an immunogenic composition comprising an PCV2b ORF2 protein to that animal in need of such treatment, wherein the immunogenic composition is subunit immunogenic composition, a compositions containing whole killed, or attenuated and/or inactivated PCV2b.

The term "subunit immunogenic composition" as used herein refers to a composition containing at least one immunogenic polypeptide or antigen, but not all antigens, derived from or homologous to an antigen from PCV2b. Such a composition is substantially free of intact PCV2b. Thus, a "subunit immunogenic composition" is prepared from at least partially purified or fractionated (preferably substantially purified) immunogenic polypeptides from PCV2b, or recombinant analogs thereof. A subunit immunogenic composition can comprise the subunit antigen or antigens of interest substantially free of other antigens or polypeptides from PCV2b, or in fractionated from. A preferred immunogenic subunit composition comprises the PCV2b ORF-2 protein as described herein.

An "immune response" means but is not limited to the development in a host of a cellular and/or antibody-mediated immune response to the composition or vaccine of interest. Usually, an "immune response" includes but is not limited to one or more of the following effects: the production or activation of antibodies, B cells, helper T cells, suppressor T cells, and/or cytotoxic T cells, directed specifically to an antigen or antigens included in the composition or vaccine of interest. Preferably, the host will display either a therapeutic or a protective immunological (memory) response such that resistance to new infection will be enhanced and/or the clinical severity of the disease reduced. Such protection will be demonstrated by either a reduction in number or severity of, or lack of one or more of the signs associated with PCV2 infections, in particular an infection with PCV2 subtype b (PCV2b) and/or an infection with PCV2 of a subtype other than subtype 2b, in delay of onset of viremia, in a reduced viral persistence, in a reduction of the overall viral load and/or a reduction of viral excretion.

The terms "antigen" as used herein refer to an amino acid sequence which elicits an immunological response as described above.

According to a further aspect, the immunogenic composition as used herein most preferably comprises the protein, or a fragment thereof, expressed by ORF-2 of PCV2b. PCV2b ORF-2 DNA and protein used herein for the preparation of the compositions and within the processes provided herein is a highly conserved domain within PCV2b isolates and thereby, any PCV2b ORF-2 would be effective as the source of the PCV2b ORF-2 DNA and/or protein as used herein. A preferred PCV2b ORF-2 protein is that of SEQ ID NO:1. However, it is understood by those of skill in the art that this sequence could vary by as much as 1-5% in sequence homology and still retain the antigenic characteristics that render it useful in immunogenic compositions according to invention.

"Sequence Identity" as it is known in the art refers to a relationship between two or more polypeptide sequences or two or more polynucleotide sequences, namely a reference sequence and a given sequence to be compared with the reference sequence. Sequence identity is determined by comparing the given sequence to the reference sequence after the sequences have been optimally aligned to produce the highest degree of sequence similarity, as determined by the match between strings of such sequences. Upon such alignment, sequence identity is ascertained on a position-by-position basis, e.g., the sequences are "identical" at a particular position if at that position, the nucleotides or amino acid residues are identical. The total number of such position identities is then divided by the total number of nucleotides or residues in the reference sequence to give % sequence identity. Sequence identity can be readily calculated by known methods, including but not limited to, those described in Computational Molecular Biology, Lesk, A. N., ed., Oxford University Press, New York (1988), Biocomputing: Informatics and Genome Projects, Smith, D.W., ed., Academic Press, New York (1993); Computer Analysis of Sequence Data, Part I, Griffin, A.M., and Griffin, H. G., eds., Humana Press, New Jersey (1994); Sequence Analysis in Molecular Biology, von Heinge, G., Academic Press (1987); Sequence Analysis Primer, Gribskov, M. and Devereux, J., eds., M. Stockton Press, New York (1991); and Carillo, H., and Lipman, D., SIAM J. Applied Math., 48: 1073 (1988), the teachings of which are incorporated herein by reference. Preferred methods to determine the sequence identity are designed to give the largest match between the sequences tested. Methods to determine sequence identity are codified in publicly available computer programs which determine sequence identity between given sequences. Examples of such programs include, but are not limited to, the GCG program package (Devereux, J., et al., Nucleic Acids Research, 12(1):387 (1984)), BLASTP, BLASTN and FASTA (Altschul, S. F. et al., J. Molec. Biol., 215:403-410 (1990). The BLASTX program is publicly available from NCBI and other sources (BLAST Manual, Altschul, S. et al., NCVI NLM NIH Bethesda, MD 20894, Altschul, S. F. et al., J. Molec. Biol., 215:403-410 (1990), the teachings of which are incorporated herein by reference). These programs optimally align sequences using default gap weights in order to produce the highest level of sequence identity between the given and reference sequences. As an illustration, by a polynucleotide having a nucleotide sequence having at least, for example, 85%, preferably 90%, even more preferably 95% "sequence identity" to a reference nucleotide sequence, it is intended that the nucleotide sequence of the given polynucleotide is identical to the reference sequence except that the given polynucleotide sequence may include up to 15, preferably up to 10, even more preferably up to 5 point mutations per each 100 nucleotides of the reference nucleotide sequence. In other words, in a polynucleotide having a nucleotide sequence having at least 85%, preferably 90%, even more preferably 95% identity relative to the reference nucleotide sequence, up to 15%, preferably 10%, even more preferably 5% of the nucleotides in the reference sequence may be deleted or substituted with another nucleotide, or a number of nucleotides up to 15%, preferably 10%, even more preferably 5% of the total nucleotides in the reference sequence may be inserted into the reference sequence. These mutations of the reference sequence may occur at the 5' or 3' terminal positions of the reference nucleotide sequence or anywhere between those terminal positions, interspersed either individually among nucleotides in the reference sequence or in one or more contiguous groups within the reference sequence. Analogously, by a polypeptide having a given amino acid sequence having at least, for example, 85%, preferably 90%, even more preferably 95% sequence identity to a reference amino acid sequence, it is intended that the given amino acid sequence of the polypeptide is identical to the reference sequence except that the given polypeptide sequence may include up to 15, preferably up to 10, even more preferably up to 5 amino acid alterations per each 100 amino acids of the reference amino acid sequence. In other words, to obtain a given polypeptide sequence having at least 85%, preferably 90%, even more preferably 95% sequence identity with a reference amino acid sequence, up to 15%, preferably up to 10%, even more preferably up to 5% of the amino acid residues in the reference sequence may be deleted or substituted with another amino acid, or a number of amino acids up to 15%, preferably up to 10%, even more preferably up to 5% of the total number of amino acid residues in the reference sequence may be inserted into the reference sequence. These alterations of the reference sequence may occur at the amino or the carboxyl terminal positions of the reference amino acid sequence or anywhere between those terminal positions, interspersed either individually among residues in the reference sequence or in the one or more contiguous groups within the reference sequence. Preferably, residue positions which are not identical differ by conservative amino acid substitutions. However, conservative substitutions are not included as a match when determining sequence identity.

"Sequence homology", as used herein, refers to a method of determining the relatedness of two sequences. To determine sequence homology, two or more sequences are optimally aligned, and gaps are introduced if necessary. However, in contrast to "sequence identity", conservative amino acid substitutions are counted as a match when determining sequence homology. In other words, to obtain a polypeptide or polynucleotide having 95% sequence homology with a reference sequence, 85%, preferably 90%, even more preferably 95% of the amino acid residues or nucleotides in the reference sequence must match or comprise a conservative substitution with another amino acid or nucleotide, or a number of amino acids or nucleotides up to 15%, preferably up to 10%, even more preferably up to 5% of the total amino acid residues or nucleotides, not including conservative substitutions, in the reference sequence may be inserted into the reference sequence. Preferably the homolog sequence comprises at least a stretch of 50, even more preferably at least 100, even more preferably at least 250, and even more preferably at least 500 nucleotides.

A "conservative substitution" refers to the substitution of an amino acid residue or nucleotide with another amino acid residue or nucleotide having similar characteristics or properties including size, hydrophobicity, etc., such that the overall functionality does not change significantly.

"Isolated" means altered "by the hand of man" from its natural state, i.e., if it occurs in nature, it has been changed or removed from its original environment, or both. For example, a polynucleotide or polypeptide naturally present in a living organism is not "isolated," but the same polynucleotide or polypeptide separated from the coexisting materials of its natural state is "isolated", as the term is employed herein.

Thus, according to a further aspect, the present invention also provides a method for the treatment or prevention of a PCV2 infection or for reduction of clinical signs caused by or associated with a PCV2 infection in animals, preferably animals having anti-PCV2 antibodies, in particular maternal derived anti-PCV2 antibodies, comprising the step of administering an effective amount of PCV2b ORF2 protein or an immunogenic composition comprising PCV2b) ORF2 protein to that animal in need of such treatment, wherein said PCV2b ORF-2 protein is anyone of those, described herein. Preferably, said PCV2 ORF-2 protein is:
i) a polypeptide comprising or consisting of the sequence of SEQ ID NO: 1 ;or
ii) any polypeptide that is at least 95% homologous to the polypeptide of i).

According to a further aspect, PCV2b ORF-2 protein is provided in the immunogenic composition at a protein inclusion level effective for inducing the desired immune response, namely reducing the incidence of, lessening the severity of, or preventing or reducing one or more clinical signs resulting from or associated with a PCV2 infection. Preferably, the PCV2b ORF-2 protein inclusion level is at least 0.2 µg protein / ml of the final immunogenic composition (µg/ml), more preferably from about 0.2 to about 400 µg/ml, still more preferably from about 0.3 to about 200 µg/ml, even more preferably from about 0.35 to about 100 µg/ml, still more preferably from about 0.4 to about 50 µg/ml, still more preferably from about 0.45 to about 30 µg/ml, still more preferably from about 0.5 to about 18 µg /ml, even more preferably from about 0.6 to about 15 µg/ml even more preferably from about 0.75 to about 8 µg/ml, even more preferably from about 1.0 to about 6 µg/ml, still more preferably from about 1.3 to about 3.0 µg/ml, even more preferably from about 1.4 to about 2.5 µg/ml, even more preferably from about 1.5 to about 2.0 µg/ml, and most preferably about 1.6 µg/ml.

According to a further aspect, the protein inclusion level is at least 0.2 µg/ PCV2b ORF-2 protein as described above per dose of the final immunogenic composition (µg/dose), more preferably from about 0.2 to about 400 µg/dose, still more preferably from about 0.3 to about 200 µg/dose, even more preferably from about 0.35 to about 100 µg/dose, still more preferably from about 0.4 to about 50 µg/dose, still more preferably from about 0.45 to about 30 µg/dose, still more preferably from about 0.5 to about 18 µg/dose, even more preferably from about 0.6 to about 15 µg/ml, even more preferably from about 0.75 to about 8 µg/dose, even more preferably from about 1.0 to about 6 µg/dose, still more preferably from about 1.3 to about 3.0 µg/dose, even more preferably from about 1.4 to about 2.5 µg/dose, even more preferably from about 1.5 to about 2.0 µg/dose, and most preferably about 1.6 µg/dose. Also, a PCV2b ORF-2 protein inclusion level (antigen content) of less than 20 µg/dose, preferably of about 0.5 to 18 µg/dose is suitable to confer immunity in young animals and/or in animals which are positive for PCV2 antibodies, in particular which are positive for anti-PCV2 maternal derived antibodies. Thus, according to a further aspect, the present invention also provides a method for the treatment or prevention of a PCV2 infection or for reduction of clinical signs caused by or associated with a PCV2 infection in animals, preferably animals having anti-PCV2 antibodies, in particular maternal derived anti-PCV2 antibodies, comprising the step of administering less than 20 µg/ dose, preferably of about 0.5 to 18 µg/dose of PCV2b ORF2 protein or an immunogenic composition comprising PCV2b ORF2 protein to that animal in need of such treatment. Said PCV2b ORF2 protein is anyone described in this patent application.

The PCV2b ORF-2 protein used in the immunogenic composition in accordance with the present invention can be derived in any fashion including isolation and purification of PCV2b ORF2, standard protein synthesis, and recombinant methodology. Preferred methods for obtaining PCV2b ORF-2 protein are provided in WO06/072065, the teachings and content of which are hereby incorporated by reference in its entirety, since surprisingly it has been found that the methods described therein for obtaining PCV2a ORF-2 polypeptide can be used accordingly for obtaining PCV2b ORF-2 protein. Briefly, susceptible cells are infected with a recombinant viral vector containing PCV2b ORF-2 DNA coding sequences, PCV2b ORF-2 protein is expressed by the recombinant virus, and the expressed PCV2 ORF-2 protein is recovered from the supernatant by filtration and inactivated by any conventional method, preferably using binary ethylenimine, which is then neutralized to stop the inactivation process.

The immunogenic composition as used herein also refers to a composition that comprises i) any of the PCV2b ORF-2 proteins described above, preferably in concentrations described above, and ii) at least a portion of the viral vector expressing said PCV2b ORF-2 protein, preferably of a recombinant baculovirus. Moreover, the immunogenic composition can comprise i) any of the PCV2b ORF-2 proteins described above, preferably in concentrations described above, ii) at least a portion of the viral vector expressing said PCV2b ORF-2 protein, preferably of a recombinant baculovirus, and iii) a portion of the cell culture supernatant.

Thus, according to a further aspect, the present invention also provides a method for the treatment or prevention of a PCV2 infection or for reduction of clinical signs caused by or associated with a PCV2 infection in animals, preferably animals having anti-PCV2 antibodies, in particular maternal derived anti-PCV2 antibodies, comprising the step of administering an effective amount of PCV2b ORF2 protein or an immunogenic composition comprising PCV2b ORF2 protein to that animal in need of such treatment, wherein the PCV2b ORF2 protein is recombinant PCV2b ORF-2, preferably a baculovirus expressed PCV2b ORF-2 protein. Preferably those recombinant or baculovirus expressed PCV2b ORF-2 protein having the sequence as described above.

The immunogenic composition as used herein also refers to a composition that comprises i) any of the PCV2b ORF-2 proteins described above, preferably in concentrations described above, ii) at least a portion of the viral vector expressing said PCV2 ORF-2 protein, preferably of a recombinant baculovirus, and iii) a portion of the cell culture; wherein about 90% of the components have a size smaller than 1 µm.

The immunogenic composition as used herein also refers to a composition that comprises i) any of the PCV2b ORF-2 proteins described above, preferably in concentrations described above, ii) at least a portion of the viral vector expressing said PCV2b ORF-2 protein, iii) a portion of the cell culture, iv) and inactivating agent to inactivate the recombinant viral vector preferably BEI, wherein about 90% of the components i) to iii) have a size smaller than 1 µm. Preferably, BEI is present in concentrations effective to inactivate the baculovirus, preferably in an amount of 2 to about 8 mM BEI, preferably of about 5 mM BEI.

The immunogenic composition as used herein also refers to a composition that comprises i) any of the PCV2b ORF-2 proteins described above, preferably in concentrations described above, ii) at least a portion of the viral vector expressing said PCV2b ORF-2 protein, iii) a portion of the cell culture, iv) an inactivating agent to inactivate the recombinant viral vector preferably BEI, and v) an neutralization agent to stop the inactivation mediated by the inactivating agent, wherein about 90% of the components i) to iii) have a size smaller than 1 µm. Preferably, if the inactivating agent is BEI, said composition comprises sodium thiosulfate in equivalent amounts to BEI.

The protein is incorporated into a composition that can be administered to an animal susceptible to PCV2 infection. In preferred forms, the composition may also include additional components known to those of skill in the art (see also Remington's Pharmaceutical Sciences. (1990). 18th ed. Mack Publ., Easton). Additionally, the composition may include one or more veterinary-acceptable carriers. As used herein, "a veterinary-acceptable carrier" includes any and all solvents, dispersion media, coatings, adjuvants, stabilizing agents, diluents, preservatives, antibacterial and antifungal agents, isotonic agents, adsorption delaying agents, and the like. In a preferred embodiment, the immunogenic composition comprises PCV2b ORF-2 protein as provided herewith, preferably in concentrations described above, which is mixed with an adjuvant, preferably Carbopol, and physiological saline.

Those of skill in the art will understand that the composition used herein may incorporate known injectable, physiologically acceptable sterile solutions. For preparing a ready-to-use solution for parenteral injection or infusion, aqueous isotonic solutions, such as e.g., saline or corresponding plasma protein solutions, are readily available. In addition, the immunogenic and vaccine compositions of the present invention can include diluents, isotonic agents, stabilizers, or adjuvants. Diluents can include water, saline, dextrose, ethanol, glycerol, and the like. Isotonic agents can include sodium chloride, dextrose, mannitol, sorbitol, and lactose, among others. Stabilizers include albumin and alkali salts of ethylendiamintetracetic acid, among others.

"Adjuvants" as used herein, can include aluminium hydroxide and aluminium phosphate, saponins e.g., Quil A, QS-21 (Cambridge Biotech Inc., Cambridge MA), GPI-0100 (Galenica Pharmaceuticals, Inc., Birmingham, AL), water-in-oil emulsion, oil-in-water emulsion, water-in-oil-in-water emulsion. The emulsion can be based in particular on light liquid paraffin oil (European Pharmacopea type); isoprenoid oil such as squalane or squalene oil resulting from theoligomerization of alkenes, in particular of isobutene or decene; esters of acids or of alcohols containing a linear alkyl group, more particularly plant oils, ethyl oleate, propylene glycol di-(caprylate/caprate), glyceryl tri-(caprylate/caprate) or propylene glycol dioleate; esters of branched fatty acids or alcohols, in particular isostearic acid esters. The oil is used in combination with emulsifiers to form the emulsion. The emulsifiers are preferably nonionic surfactants, in particular esters of sorbitan, of mannide (e.g. anhydromannitol oleate), of glycol, of polyglycerol, of propylene glycol and of oleic, isostearic, ricinoleic or hydroxystearic acid, which are optionally ethoxylated, and polyoxypropylenepolyoxyethylene copolymer blocks, in particular the Pluronic products, especially L121. See Hunter et al., The Theory and Practical Application of Adjuvants (Ed.Stewart-Tull, D. E. S.). JohnWiley and Sons, NY, pp51-94 (1995) and Todd et al., Vaccine 15:564-570 (1997).

For example, it is possible to use the SPT emulsion described on page 147 of "Vaccine Design, The Subunit and Adjuvant Approach" edited by M. Powell and M. Newman, Plenum Press, 1995, and the emulsion MF59 described on page 183 of this same book.

A further instance of an adjuvant is a compound chosen from the polymers of acrylic or methacrylic acid and the copolymers of maleic anhydride and alkenyl derivative. Advantageous adjuvant compounds are the polymers of acrylic or methacrylic acid which are cross-linked, especially with polyalkenyl ethers of sugars or polyalcohols. These compounds are known by the term carbomer (Pharmeuropa Vol. 8, No. 2, June 1996). Persons skilled in the art can also refer to U. S. Patent No. 2,909,462 which describes such acrylic polymers cross-linked with a polyhydroxylated compound having at least 3 hydroxyl groups, preferably not more than 8, the hydrogen atoms of at least three hydroxyls being replaced by unsaturated aliphatic radicals having at least 2 carbon atoms. The preferred radicals are those containing from 2 to 4 carbon atoms, e.g. vinyls, allyls and other ethylenically unsaturated groups. The unsaturated radicals may themselves contain other substituents, such as methyl. The products sold under the name Carbopol ; (BF Goodrich, Ohio, USA) are particularly appropriate. They are cross-linked with an allyl sucrose or with allyl pentaerythritol. Among them, there may be mentioned Carbopol 974P, 934P and 971P. Most preferred is the use of Carbopol, in particular the use of Carbopol 971P, preferably in amounts of about 500 µg to about 5 mg per dose, even more preferred in an amount of about 750 µg to about 2.5 mg per dose and most preferred in an amount of about 1 mg per dose.

Further suitable adjuvants include, but are not limited to, the RIBI adjuvant system (Ribi Inc.), Block co-polymer (CytRx, Atlanta GA), SAF-M (Chiron, Emeryville CA), monophosphoryl lipid A, Avridine lipid-amine adjuvant, heat-labile enterotoxin from E. coli (recombinant or otherwise), cholera toxin, IMS 1314, or muramyl dipeptide among many others.

Preferably, the adjuvant is added in an amount of about 100 µg to about 10 mg per dose. Even more preferably, the adjuvant is added in an amount of about 100 µg to about 10 mg per dose. Even more preferably, the adjuvant is added in an amount of about 500 µg to about 5 mg per dose. Even more preferably, the adjuvant is added in an amount of about 750 µg to about 2.5 mg per dose. Most preferably, the adjuvant is added in an amount of about 1 mg per dose.

Additionally, the composition can include one or more pharmaceutical-acceptable carriers. As used herein, "a pharmaceutical-acceptable carrier" includes any and all solvents, dispersion media, coatings, stabilizing agents, diluents, preservatives, antibacterial and antifungal agents, isotonic agents, adsorption delaying agents, and the like. Most preferably, the composition provided herewith, contains PCV2b ORF-2 protein recovered from the supernatant of in vitro cultured cells, wherein said cells were infected with a recombinant viral vector containing PCV2b ORF-2 DNA and expressing PCV2b ORF-2 protein, and wherein said cell culture was treated with about 2 to about 8 mM BEI, preferably with about 5 mM BEI to inactivate the viral vector, and an equivalent concentration of a neutralization agent, preferably sodium thiosulfate solution to a final concentration of about 2 to about 8 mM, preferably of about 5 mM.

The present invention also relates to an immunogenic composition that comprises i) any of the PCV2b ORF-2 proteins described above, preferably in concentrations described above, ii) at least a portion of the viral vector expressing said PCV2b ORF-2 protein, iii) a portion of the cell culture, iv) an inactivating agent to inactivate the recombinant viral vector preferably BEI, and v) an neutralization agent to stop the inactivation mediated by the inactivating agent, preferably sodium thiosulfate in equivalent amounts to BEI; and vi) a suitable adjuvant, preferably Carbopol 971 in amounts described above; wherein about 90% of the components i) to iii) have a size smaller than 1 µm. According to a further aspect, this immunogenic composition further comprises a pharmaceutical acceptable salt, preferably a phosphate salt in physiologically acceptable concentrations. Preferably, the pH of said immunogenic composition is adjusted to a physiological pH, meaning between about 6.5 and 7.5.

The immunogenic composition as used herein also refers to a composition that comprises per one ml i) at least 1.6 µg of PCV2b ORF-2 protein described above, preferably less than 20 µg ii) at least a portion of baculovirus expressing said PCV2b ORF-2 protein iii) a portion of the cell culture, iv) about 2 to 8 mM BEI, v) sodium thiosulfate in equivalent amounts to BEI; and vi) about 1 mg Carbopol 971, and vii) phosphate salt in a physiologically acceptable concentration; wherein about 90% of the components i) to iii) have a size smaller than 1 µm and the pH of said immunogenic composition is adjusted to about 6.5 to 7.5.

The immunogenic compositions can further include one or more other immunomodulatory agents such as, e.g., interleukins, interferons, or other cytokines. The immunogenic compositions can also include Gentamicin and Merthiolate. While the amounts and concentrations of adjuvants and additives useful in the context of the present invention can readily be determined by the skilled artisan, the present invention contemplates compositions comprising from about 50 µg to about 2000 µg of adjuvant and preferably about 250 µg/ ml dose of the vaccine composition. Thus, the immunogenic composition as used herein also refers to a composition that comprises from about 1ug/ml to about 60 µg/ml of antibiotics, and more preferably less than about 30 µg/ml of antibiotics.

The immunogenic composition as used herein also refers to a composition that comprises i) any of the PCV2b ORF-2 proteins described above, preferably in concentrations described above, ii) at least a portion of the viral vector expressing said PCV2b ORF-2 protein, iii) a portion of the cell culture, iv) an inactivating agent to inactivate the recombinant viral vector preferably BEI, and v) an neutralization agent to stop the inactivation mediated by the inactivating agent, preferably sodium thiosulfate in equivalent amounts to BEI; vi) a suitable adjuvant, preferably Carbopol 971 in amounts described above; vii) a pharmaceutical acceptable concentration of a saline buffer, preferably of a phosphate salt, and viii) an antimicrobiological active agent; wherein about 90% of the components i) to iii) have a size smaller than 1 µm.

For investigation of a possible interference of PCV2b ORF2 protein with the maternal antibody a study may be conducted in which the antibody titers of study animals are determined at the time of vaccination which are then grouped into a low, moderate and high antibody class: Geometric mean titers of < 1:100 are considered as low antibody titers, titers of 1:100 to 1:1000 are considered as moderate antibody titers and titers of >1:1000 are considered as high antibody titers. This grouping pattern is comparable to that done in a Canadian field study where antibody titers of 1:80 were considered as low, antibody titers of 1:640 as moderate and antibody titers of > 1:1280 as high (Larochelle et al., 2003, Can. J. Vet. Res.; 67: 114-120). In order to analyze the impact of low, medium and high antibody titers at the time of vaccination on viremia, vaccinated and placebo-treated animals are compared with regard to the onset, end, duration of viremia, the number of positive sampling days and the virus load. The presence of anti-PCV2 antibodies, in particular of maternal derived antibodies, preferably has no significant impact of any of those parameters. In other words, the efficacy of the PCV2b ORF2 protein in prevention and treatment of a PCV2 infection or in reduction of clinical signs caused by or associated with a PCV2 infection in animals is preferably not affected at the day of vaccination by the presence of anti-PCV2 antibodies, preferably by anti-PCV2 antibody titers of up to 1:100, preferably of more than 1:100, even more preferably of more than 1:250, even more preferably of more than 1:500, even more preferably of 1:640; even more preferably of more than 1:750, most preferably of more than 1:1000. This effect can be shown in a one shot vaccination experiment, which means that the PCV2b ORF2 protein is administered only once and without any subsequent administration of PCV2b ORF2 protein.

Methods for detection and quantification of anti-PCV2 antibodies are well known in the art. For example detection and quantification of PCV2 antibodies can be performed by indirect immunofluorescence as described in Magar et al., 2000, Can. J. Vet Res. ; 64: 184-186 or Magar et al., 2000, J. Comp. Pathol.; 123: 258-269. Further assays for quantification of anti-PCV2 antibodies are described in Opriessnig et al., 2006, 37th Annual Meeting of the American Association of Swine Veterinarians. Moreover, an indirect immunofluorescence assay, that can be used by a person skilled in the art comprises the steps of: (i) seeding about 20.000 to 60.000 PK15 or VIDO R1 cells per well onto a 96 well plate, (ii) infecting cells with a PCV2 isolate, when monolayers are approximately 65 to 85% confluent, (iii) incubating infected cells for 48 hours, (iv) removing medium and washing cells 2 times with PBS, (v) discarding the wash buffer and treating cells with cold 50/50 methanol/acetone fixative (-100 µl/well) for about 15 min at about -20°C, (vi) discarding the fixative and air drying of the plates,(vii) preparing serial dilutions of porcine serum samples in PBS and serial dilutions of an anti-PCV2 positive and negative control sample (Positive Control and Negative Control Samples), (viii) adding the serial dilutions to the plates and incubating to allow antibodies to bind if present in the serum samples for about 1 hr at 36,5±1°C, (ix) washing the plates three times with PBS an discarding the PBS, (x) staining the plates with a commercial Goat anti-Swine FITC conjugate diluted 1:100 in PBS and incubated for about 1 hr. at 36.5±1°C, (xi) removing microplates are removed from incubator, the conjugate is discarded and the plates are washed 2 times with PBS, (xii) reading the plates using UV microscopy and reporting individual wells as positive or negative, wherein the Positive Control and Negative Control samples are used to monitor the test system, (xiii)calculating the serum antibody titers using the highest dilution showing specific IFA reactivity and the number of wells positive per dilution, or a 50% endpoint is calculated using the appropriate Reed-Muench formula.

Such an assay is described in Example 2 of WO 2008/076915 A2.

In cases of controversial results and in any question of doubt, anti-PCV2 titers as mentioned herein, refer to those which are/can be estimated by this assay.

Thus according to a further aspect, the present invention provides a method for the treatment or prevention of a PCV2 infection or for reduction of clinical signs caused by or associated with a PCV2 infection in animals, preferably animals having anti-PCV2 antibodies, in particular maternal antibodies, comprising the step of administering an effective amount of a PCV2b ORF2 protein to that animal in need of such treatment, preferably of less than 20 µg/dose wherein said animal have a detectable anti-PCV2 antibody titer of up to 1:100, preferably of more than 1:100, even more preferably of more than 1:250, even more preferably of more than 1:500, even more preferably of 1:640, even more preferably of more than 1:750, most preferably of more than 1:1000. Preferably, the aforementioned anti-PCV2 antibody titers are detectable and quantifiable in a specific anti-PCV2 immune assay, preferably in the assay as described above, as exemplarily described in Example 2 of WO 2008/076915 A2. More preferably, anti-PCV-2 antibodies are maternal derived antibodies. Most preferably, the PCV2b ORF2 protein is only administered once, preferably with a dose of less than 20 µg/dose.

Piglets with only low titers (< 1:100) or moderate titers (< 1:1000) of maternal derived anti-PCV2 antibodies are not sufficient protected against PCV2 infections which occur prior to week 3 of age. Therefore, vaccination at a very early stage of life is desirable. Within the context of the invention, vaccination/treatment of animals at or before 3 weeks of age is preferred. Moreover, anti-PCV2 antibody titers of more than 1:1000 preferably have no influence on the efficacy of the PCV2 vaccine regardless of the level of the existing initial antibody titer. For example, vaccination of high-titer animals (anti-PCV2 antibody titer > 1:1000) preferably result in a shorter duration of viremia, an earlier end of viremia, less viremic sampling days and a reduction of the sum of genomic equivalents/ml as compared to non vaccinated control animals. Upon comparison of vaccinated "high", "moderate" and "low titer animals" no significant differences are preferably observed with regard to the different parameters of PCV2 viraemia. Also in the presence of high anti-PCV2 antibody titers the PCV2b ORF2 protein used for vaccination preferably still significantly reduces viremia in blood (end of viremia, duration of viremia, virus load). Preferably, no differences are found with regard to the live body weight when comparing low and high titer animals of the vaccinated group. Furthermore, vaccinated animals with a high anti-PCV2 antibody titer at the time of vaccination/treatment (> 1:1000) also preferably show a significantly higher body weight after the onset of viremia compared to placebo-treated animals with initial high antibody titers. Consequently, according to a preferred aspect, vaccination/treatment of animals of 1 day of age or older with PCV2b ORF2 protein is possible. However, vaccination should be done within the first 8, preferably within the first 7 weeks of age. Thus according to a further aspect, the present invention provides a method for the treatment or prevention of a PCV2 infection or for reduction of clinical signs caused by or associated with a PCV2 infection in animals, comprising the step of administering to that animal in need of such treatment at day 1 of age or later, preferably but not later than at week 8 of age an effective amount of a PCV2b ORF2 protein. According to a preferred embodiment, less than 20 µg/dose PCV2b ORF2 protein are required to confer immunity in such animal. According to a more preferred embodiment, the PCV2b ORF2 protein, preferably less than 20 µg/dose thereof is only administered once to the animal in need of such treatment.

According to a further, more general aspect, the present invention provides a method for the treatment or prevention of a PCV2 infection or for reduction of clinical signs caused by or associated with a PCV2 infection in young animals, comprising the step of administering an effective amount of a PCV2b ORF2 protein to that animal in need of such treatment.

The term "young animal" as used herein refers to an animal of 1 to 22 days of age. Preferably, by the term young animal, an animal of 1 to 20 days of age is meant. More preferably, the term young animal refers to an animal of 1 to 15 days of age, even more preferably of 1 day of age to 14 days of age, even more preferably of 1 to 12 days of age, even more preferably of 1 to 10 days of age, even more preferably of 1 to 8 days of age, even more preferably of 1 to 7 days of age, even more preferably of 1 to 6 days of age, even more preferably of 1 to 5 days of age, even more preferably of 1 to 4 days of age, even more preferably of 1 to 3 days of age, even more preferably of 1 or 2 day(s) of age, most preferably to an animal of 1 day of age. Thus according to a further aspect, the present invention provides a method for the treatment or prevention of a PCV2 infection or for reduction of clinical signs caused by or associated with a PCV2 infection in young animals, comprising the step of administering an effective amount of a PCV2b ORF2 protein to an animal of 1 to 22 days of age, preferably of 1 to 20 days of age, more preferably of 1 to 15 days of age, even more preferably of 1 to 14 days of age, even more preferably of 1 to 12 days of age, even more preferably of 1 to 10 days of age, even more preferably of 1 to 8 days of age, even more preferably of 1 to 7 days of age, even more preferably of 1 to 6 days of age, even more preferably of 1 to 5 days of age, even more preferably of 1 to 4 days of age, even more preferably of 1 to 3 days of age, even more preferably of 1 or 2 day(s) of age, most preferably at 1 day of age in need of such treatment. For example, the vaccination/treatment on 19 to 22 days of age preferably shows high efficacy of vaccination. Moreover, vaccination/treatment at 12 to 18, preferably 12 to 14 days of age is preferably very effective in reduction of clinical signs associated with PCV2 infections, reduction of overall viral load, reduction of duration of viremia, delay in onset of viremia, weight gain. Moreover, vaccination at 1 week of age is preferably very effective in reduction of clinical signs associated with PCV2 infections, reduction of overall viral load, reduction of duration of viremia, delay in onset of viremia, weight gain. Preferably less than 20 µg/dose PCV2b ORF2 protein is required to confer immunity in those young animals. According to a more preferred embodiment, the PCV2b ORF2 protein, preferably less than 20 µg is only administered once to that young animal in need of such treatment.

Due to the ubiquity of PCV2 in the field most of the young piglets are seropositive in respect to PCV2. Thus according to a further aspect, the present invention provides a method for the treatment or prevention of a PCV2 infection or for reduction of clinical signs caused by or associated with a PCV2 infection in young animals, preferably animals having anti-PCV2 antibodies at the day of vaccination, comprising the step of administering an effective amount of a PCV2b ORF2 protein to an animal of 1 to 22 days of age, preferably of 1 to 20 days of age, more preferably of 1 to 15 days of age, even more preferably of 1 to 14 days of age, even more preferably of 1 to 12 days of age, even more preferably of 1 to 10 days of age, even more preferably of 1 to 8 days of age, even more preferably of 1 to 7 days of age, even more preferably of 1 to 6 days of age, even more preferably of 1 to 5 days of age, even more preferably of 1 to 4 days of age, even more preferably of 1 to 3 days of age, even more preferably at 1 or 2 day(s) of age, most preferably at 1 day of age in need of such treatment. Preferably, said young animals, at the day of vaccination/treatment, have a detectable anti-PCV2 antibody titer of up to 1:100, preferably of more than 1:100, even more preferably of more than 1:250, even more preferably of more than 1:500, even more preferably of 1:640, even more preferably of more than 1:750, most preferably of more than 1:1000 at the day of vaccination/treatment. Preferably less than 20 µg/dose PCV2b ORF2 protein are required to confer a sufficient immunity in those young animals. According to more preferred embodiment, the PCV2b ORF2 protein, preferably less than 20 µg is only administered once to that young animal in need of such treatment.

As described above, vaccination/treatment of young animals with PCV2b ORF2 protein preferably results in shortening of viremic phase as compared to non vaccinated control animals. The average shortening time may preferably, for instance, be 9.5 days as compared to non vaccinated control animals of the same species. Therefore, according to a further aspect, the present invention also provides a method for the treatment or prevention of a PCV2 infection or for reduction of clinical signs caused by or associated with a PCV2 infection in young animals, comprising the step of administering an effective amount of a PCV2b ORF2 protein to that animal in need of such treatment, wherein the treatment or prevention results in shortening of the viremia phase of 5 or more days, preferably 6 or more days, even more preferably of 7 or more days, even more preferably of 8 or more days, even more preferably of 9, even more preferably of 10, even more preferably of 12, even more preferably of 14, most preferably of more than 16 days as compared to animals of a non-treated control group of the same species. In some cases, the viremic phase is preferably shortening for more than 20 days. In general, the vaccination of young piglets preferably results in a reduction in the loss of weight gain, a shorter duration of viremia, an earlier end to viremia, and a lower virus load. Therefore, according to a further aspect, the present invention provides a method for the treatment or prevention of a PCV2 infection or for reduction of clinical signs caused by or associated with a PCV2 infection in young animals, comprising the step of administering an effective amount of a PCV2b ORF2 protein to that animal in need of such treatment, wherein said treatment or prevention of PCV2 infection results in an improvement in comparison to animals of a non-treated control group of the same species in a vaccine efficacy parameter selected from the group consisting of a reduction in the loss of weight gain, a shorter duration of viremia, an earlier end to viremia, a lower virus load, or combinations thereof. Preferably less than 20 µg/dose PCV2b ORF2 protein are required cause any of the improved vaccine efficacy parameter mentioned above. Moreover such improved vaccine efficacy parameter are achieved by a singly administration of only one dose.

The term "an effective amount" as used herein means but is not limited to an amount of PCV2b ORF2 protein, that elicits or is able to elicit an immune response in an animal, to which said effective dose of PCV2b ORF2 protein is administered. Preferably, an effective amount is defined as an amount of PCV2b ORF2 protein that confers at least a 10 weeks duration of immunity (DOI), preferably at least a12 weeks (DOI), more preferably at least a 15 weeks (DOI), most preferably at least a 20 weeks (DOI).

The amount that is effective depends on the ingredients of the vaccine and the schedule of administration. Typically, when an inactivated virus or a modified live virus preparation is used in the combination vaccine, an amount of the vaccine containing about 10^{2.0} to about 10^{9.0} TCID₅₀ per dose, preferably about 10^{3.0} to about 10^{8.0} TCID₅₀ per dose, more preferably, about 10^{4.0} to about 10^{8.0} TCID₅₀ per dose. In particular, when modified live PCV2b is used in the vaccines, the recommended dose to be administered to the susceptible animal is preferably about 10^{3.0} TCID₅₀ (tissue culture infective dose 50% end point)/dose to about 10^{6.0} TCID₅₀/dose and more preferably about 10^{4.0} TCID₅₀/dose to about 10^{5.0} TCID₅₀/dose. In general, the quantity of antigen will be between 0.2 and 5000 micrograms, and between 10^{2.0} and 10^{9.0} TCID₅₀, preferably between 10^{3.0} and 10^{6.0} TCID₅₀, more preferably between 10^{4.0} and 10^{5.0} TCID₅₀, when purified antigen is used.

Sub-unit vaccines are normally administered with an protein inclusion level of at least 0.2 µg protein per dose, preferably with about 0.2 to about 400 µg/dose, still more preferably with about 0.3 to about 200 µg/dose, even more preferably with about 0.35 to about 100 µg/dose, still more preferably with about 0.4 to about 50 µg/dose, still more preferably with about 0.45 to about 30 µg/dose, still more preferably with about 0.5 to about 18 µg/dose, still more preferably with about 0.6 to about 16 µg/dose, even more preferably with about 0.75 to about 8 µg/dose, even more preferably with about 1.0 to about 6 µg/dose, still more preferably with about 1.3 to about 3.0 µg/dose.

Preferably, the prophylactic use of the immunogenic compositions described *supra,* is effective for reduction of clinical signs caused by or associated with PCV2 infections, preferably in young animals and/or in animals having passive immunity against PCV2 at the day of treatment. In particular, the prophylactic use of the immunogenic compositions as described herein, and specifically of compositions comprising PCV2b ORF-2 protein, is preferably effective for reducing lymphadenopathy, lymphoid depletion and/or multinucleated/giant histiocytes in animals infected with PCV2 and having maternal anti-PCV-2 antibodies at the day of treatment/vaccination. For example it was discovered that the prophylactic use of the immunogenic compositions as described herein is effective for reducing lymphoid depletion, lymphoid inflammation, positive IHC for PCV2 antigen of lymphoid tissue, viremia, nasal shedding, pyrexia, reduced average daily weight gain, lung inflammation, positive IHC for PCV2 antigen of lung tissue, increased mortality. Furthermore, the prophylactic use of the immunogenic compositions as described herein is preferably effective for reducing (1) interstitial pneumonia with interlobular edema, (2) cutaneous pallor or icterus, (3) mottled atrophic livers, (4) gastric ulcers , (5) nephritis and (6) reproductive disorders, e.g. abortion, stillbirths, mummies, etc, (7) Pia like lesions, normally known to be associated with Lawsonia intracellularis infections (Ileitis), (8) lymphadenopathy, (9) lymphoid depletion and/or (10) multinucleated/giant histiocytes (11) Porcine Dermatitis and Nephropathy Syndrome (PDNS), (12) PCVAD associated mortality, (13) PCVAD associated weight loss, (14), reduced growth variability (15), reduced frequency of 'runts' (16), reduced co-infections with Porcine Reproductive and Respiratory Disease Complex (PRRSV). Such immunogenic composition is also effective in improving economical important growth parameters such as time to slaughter, carcass weight, lean meat ratio. Thus the term "clinical signs" as used herein, means, but is not limited to (1) interstitial pneumonia with interlobular edema, (2) cutaneous pallor or icterus, (3) mottled atrophic livers, (4) gastric ulcers, (5) nephritis and (6) reproductive disorders, e.g. abortion, stillbirths, mummies, etc, (7) Pia like lesions, normally known to be associated with Lawsonia intracellularis infections (Ileitis), (8) lymphadenopathy, (9) lymphoid depletion and/or (10) multinucleated/giant histiocytes (11) Porcine Dermatitis and Nephropathy Syndrome (PDNS), (12) PCVAD associated mortality, (13) PCVAD associated weight loss, (14) reduced growth variability (15) reduced frequency of 'runts' (16) reduced co-infections with Porcine Reproductive and Respiratory Disease Complex (PRRSV), (17) lymphoid inflammation, (18) positive IHC for PCV2 antigen of lymphoid tissue, (19) viremia, (20) nasal shedding, (21) pyrexia, (22) reduced average daily weight gain, (23) lung inflammation, (24) positive IHC for PCV2 antigen of lung tissue, (25) increased mortality. Moreover, the immunogenic composition described herein reduces the overall circovirus load including a later onset, a shorter duration, an earlier end of viremia, and a reduced viral load and its immunosuppressive impact in young animals, in particular in those having anti-PCV2 antibodies at the day of vaccination, thereby resulting in a higher level of general disease resistance and a reduced incidence of PCV2 associated diseases and clinical signs.

Thus, according to a further aspect, the present invention provides a method for the treatment or prevention of a PCV2 infection or for reduction of clinical signs caused by or associated with a PCV2 infection in young animals and/or in animals, preferably animals having anti-PCV2 antibodies, comprising the step of administering an effective amount of PCV2b ORF2 protein or an immunogenic composition comprising an PCV2b ORF2 protein to that animal in need of such treatment, wherein those clinical signs are selected from the group consisting of: (1) interstitial pneumonia with interlobular edema, (2) cutaneous pallor or icterus, (3) mottled atrophic livers, (4) gastric ulcers , (5) nephritis and (6) reproductive disorders, e.g. abortion, stillbirths, mummies, etc, (7) Pia like lesions, normally known to be associated with Lawsonia intracellularis infections (Ileitis), (8) lymphadenopathy, (9) lymphoid depletion and/or (10) multinucleated/giant histiocytes (11), Porcine Dermatitis and Nephropathy Syndrome (PDNS), (12) PCVAD associated mortality, (13) PCVAD associated weight loss, (14) reduced growth variability (15) reduced frequency of 'runts', (16) reduced co-infections with Porcine Reproductive and Respiratory Disease Complex (PRRSV), (17) lymphoid inflammation, (18) positive IHC for PCV2 antigen of lymphoid tissue, (19) viremia, (20) nasal shedding, (21) pyrexia, (22) reduced average daily weight gain, (23) lung inflammation, (24) positive IHC for PCV2 antigen of lung tissue, (25) increased mortality. According to a further aspect, the present invention provides a method for the treatment or prevention of a PCV2 infection or for reduction of clinical signs caused by or associated with a PCV2 infection in young animals, comprising the step of administering an effective amount of a PCV2b ORF2 protein to that animal in need of such treatment, wherein those clinical signs are selected from the group consisting of: (1) interstitial pneumonia with interlobular edema, (2) cutaneous pallor or icterus, (3) mottled atrophic livers, (4) gastric ulcers , (5) nephritis and (6) reproductive disorders, e.g. abortion, stillbirths, mummies, etc, (7) Pia like lesions, normally known to be associated with Lawsonia intracellularis infections (Ileitis), (8) lymphadenopathy, (9) lymphoid depletion and/or (10) multinucleated/giant histiocytes (11) Porcine Dermatitis and Nephropathy Syndrome (PDNS), (12) PCVAD associated mortality, (13) PCVAD associated weight loss, (14) reduced growth variability (15) reduced frequency of 'runts' (16) reduced co-infections with Porcine Reproductive and Respiratory Disease Complex (PRRSV), (17) lymphoid inflammation, (18) positive IHC for PCV2 antigen of lymphoid tissue, (19) viremia, (20) nasal shedding, (21) pyrexia, (22) reduced average daily weight gain, (23) lung inflammation, (24) positive IHC for PCV2 antigen of lung tissue, (25) increased mortality.

The composition according to the invention may be applied, orally, intradermally, intratracheally, or intravaginally. The composition preferably may be applied intramuscularly or intranasally, most preferably intramuscularly. In an animal body, it can prove advantageous to apply the pharmaceutical compositions as described above via an intravenous or by direct injection into target tissues. For systemic application, the intravenous, intravascular, intramuscular, intranasal, intra-arterial, intraperitoneal, oral, or intrathecal routes are preferred. A more local application can be effected subcutaneously, intradermally, intracutaneously, intracardially, intralobally, intramedullarly, intrapulmonarily or directly in or near the tissue to be treated (connective-, bone-, muscle-, nerve-, epithelial tissue). Depending on the desired duration and effectiveness of the treatment, the compositions according to the invention may be administered once or several times, also intermittently, for instance on a daily basis for several days, weeks or months and in different dosages.

Preferably, one dose of the immunogenic composition as described above is intramuscularly administered to the subject in need thereof. According to a further aspect, the PCV2b ORF2 protein or the immunogenic composition comprising any such PCV2b ORF2 protein as described herein is bottled in and administered at one (1) mL per dose. Thus, according to a further aspect, the present invention also provides a 1 ml immunogenic composition, comprising PCV2b ORF2 protein as described herein, for the treatment or prevention of a PCV2 infection or for reduction of clinical signs caused by or associated with a PCV2 infection in young animals, comprising the step of administering an effective amount of a PCV2b ORF2 protein to that animal in need of such treatment. According to a further aspect, the present invention also provides a 1 ml immunogenic composition, comprising PCV2b ORF2 protein as described herein, treatment or prophylaxis of a PCV2 infection or for reduction of clinical signs caused by or associated with a PCV2 infection in animals, preferably animals having anti-PCV2 antibodies, comprising the step of administering an effective amount of PCV2b ORF2 protein or an immunogenic composition comprising an PCV2b ORF2 protein to that animal in need of such treatment.

According to a further aspect, at least one further administration of at least one dose of the immunogenic composition as described above is given to a subject in need thereof, wherein the second or any further administration is given at least 14 days beyond the initial or any former administrations. Preferably, the immunogenic composition is administered with an immune stimulant. Preferably, said immune stimulant is given at least twice. Preferably, at least 3 days, more preferably at least 5 days, even more preferably at least 7 days are in between the first and the second or any further administration of the immune stimulant. Preferably, the immune stimulant is given at least 10 days, preferably 15 days, even more preferably 20, even more preferably at least 22 days beyond the initial administration of the immunogenic composition provided herein. A preferred immune stimulant is, for example, keyhole limpet hemocyanin (KLH), preferably emulsified with incomplete Freund's adjuvant (KLH/ICFA). However, it is herewith understood, that any other immune stimulant known to a person skilled in the art can also be used. The term "immune stimulant" as used herein, means any agent or composition that can trigger the immune response, preferably without initiating or increasing a specific immune response, for example the immune response against a specific pathogen. It is further instructed to administer the immune stimulant in a suitable dose. The following examples set forth preferred materials and procedures in accordance with the present invention. Although any methods and materials similar or equivalent to those described herein can be used in the practice or testing of the present invention, the preferred methods, devices, and materials are now described. It is to be understood, however, that these examples are provided by way of illustration only, and nothing therein should be deemed a limitation upon the overall scope of the invention.

### EXAMPLE 1

### Preparation of PCV2b ORF-2 protein

Initial SF+ cell cultures from liquid nitrogen storage were grown Sf-900 III Serum-free Insect Medium (Invitrogen Cat#12658-027), in suspension in sterile spinner flasks with constant agitation. The cultures were grown in 100 mL to 250mL spinner flasks with 25 to 150 mL Sf-900 III Serum-free Insect Medium. When the cells had multiplied to a cell density of 4.0 - 8.0 x 10⁶ cells/mL, they were split to new vessels with a planting density of 2.0 x 10⁶ cells/mL. Subsequent expansion cultures were grown in spinner flasks up to 36 liters in size or in stainless steel bioreactors of up to 300 liters for a period of 2-7 days at 25 - 29°C.

After seeding, the flasks were incubated at 28°C for> 1 hour. Subsequently, each flask was seeded with a recombinant baculovirus containing the PCV2b ORF-2 gene **(SEQ ID NO:3).** The recombinant baculovirus containing the PCV2b ORF-2 gene was generated in accordance with the description of WO06/072065. After being seeded with the baculovirus, the flasks were then incubated at 27 ± 2°C for 4 days and were also agitated at 100 rpm during that time. The flasks used ventilated caps to allow for air flow. After incubation, the resulting supernatant was harvested, filtered in order to remove cell debris and inactivated. The supernatant was inactivated by bringing its temperature to 37 ± 2°C and binary ethlylenimine (BEI) is added to the supernatant to a final concentration of 5mM. The samples were then stirred continuously for 72 to 96 hrs. A 1.0 M sodium thiosulfate solution to give a final minimum concentration of 5 mM was added to neutralize any residual BEI. After inactivation, PCV2b ORF-2 buffered with phosphate buffer and Carbopol was added to about 0.5 to 2.5 mg/dose. The final dose comprises about 16 µg PCV2 ORF-2b protein.

### EXAMPLE 2

This study was an efficacy evaluation of an experimental Porcine Circovirus Type 2 ORF2b Vaccine against a PCV2 ORF2a and PCV2 ORF2b challenge. Sixty cesareanderived colostrum-deprived (CDCD) piglets at seven weeks of age were used in this study and separated into 4 groups; 1) 15 pigs vaccinated with an experimental Porcine Circovirus Vaccine, Type 2b, Killed Baculovirus Vector that were challenged with virulent PCV2a, 2) 15 non-vaccinated challenged control pigs that were challenged with virulent PCV2a, 3) 15 pigs vaccinated with an experimental Porcine Circovirus Vaccine, Type 2b, Killed Baculovirus Vector that were challenged with virulent PCV2b and, 4) 15 non-vaccinated challenged control pigs that were challenged with virulent PCV2b. On Day 0, Groups 1 and 3 pigs were administered 1 mL of vaccine intramuscularly (IM) whereas Groups 2 and 4, non-vaccinated challenge control pigs did not receive any treatment. On Day 28, all pigs in groups 1 and 2 were challenged with virulent PCV2-ORF2a 1mL intranasally (IN) and 1mL IM with an approximate dosage of 5.0 Log₁₀TCID₅₀/mL of live virus. On Day 28, all pigs in groups 3 and 4 were challenged with virulent PCV2-ORF2b 1mL intranasally (IN) and 1mL IM with an approximate dosage of 3.0 Log₁₀TCID₅₀/mL of live virus. All pigs received 2.0 mL Keyhole Limpet Hemocyanin emulsified in Incomplete Freunds Adjuvant (KLH/ICFA) IM on Days 25 and 31. Pigs were monitored daily for clinical signs, and blood was drawn for serologic testing periodically. On Day 56 all pigs were necropsied and select tissues were collected and gross pathology observations made.

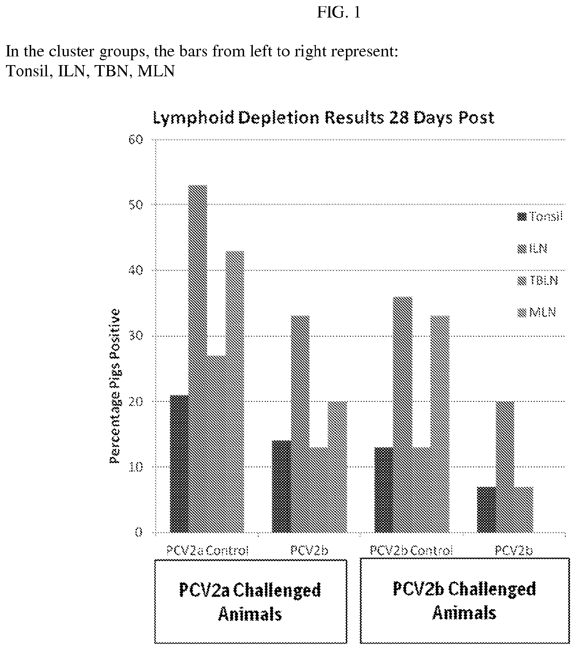

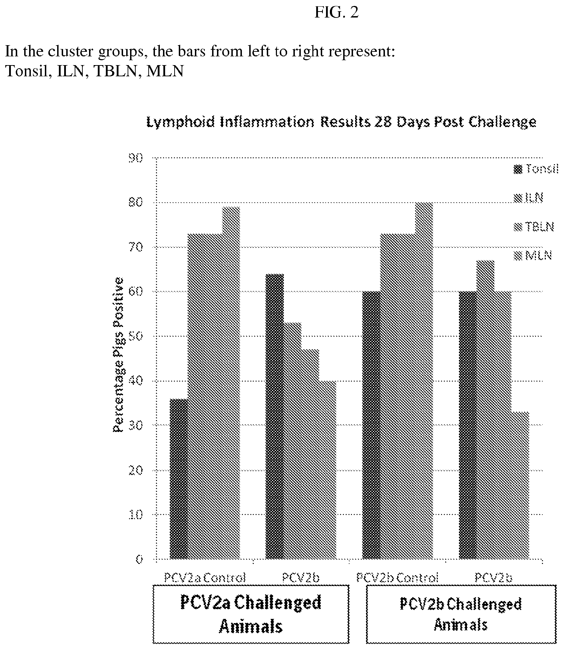

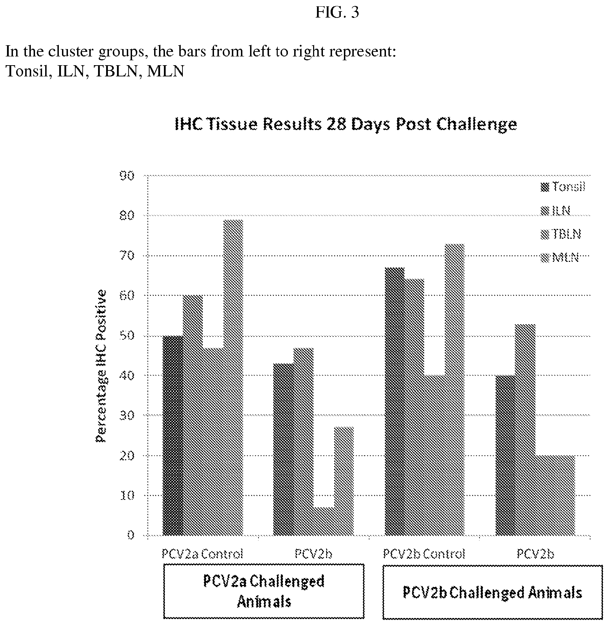

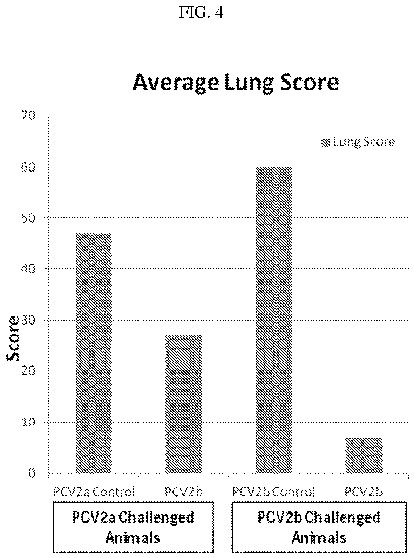

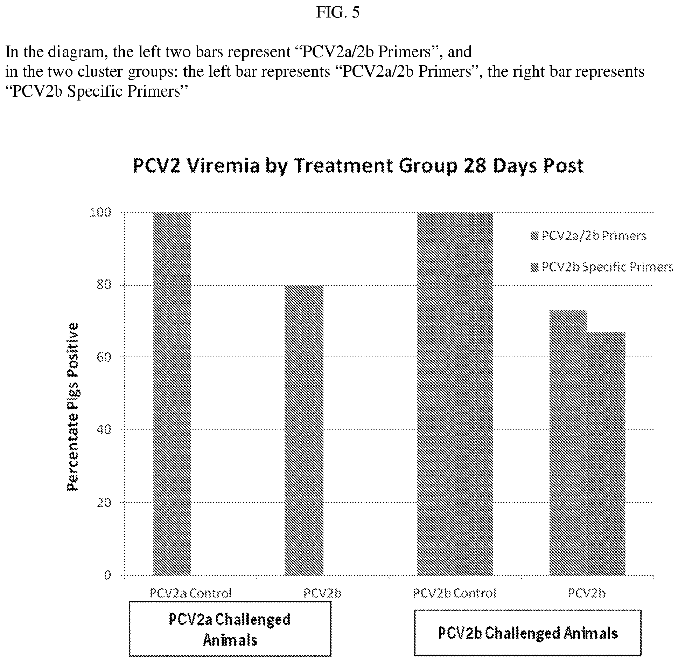

PCV-PCV2b vaccinated animals exhibited reduction when compared to their respective challenge control group in all parameters tested. Mesenteric and tracheobronchial lymph nodes showed the greatest reduction for the parameters tested. Less of a reduction was seen with iliac lymph node in the parameters tested. Tonsil samples also showed a reduction with the exception of an increase in lymphoid inflammation when comparing PCV2b vaccinated animals verses non-vaccinated controls that were challenged with PCV2a. Lung inflammation scores showed a decrease when comparing PCV2b vaccinated animals to both their respective PCV2b and PCV2a non-vaccinated controls as well. When comparing viremia on Day 56 (4 weeks post challenge) PCV2b vaccinated animals had 20% fewer viremic animals when challenged with PCV2a and an average of 30% fewer viremic animals when challenged with PCV2b.The data obtained from this study, which are also represented in Figures 1-5, indicate that a PCV2-ORF2b vaccine reduces infection against piglets exposed to both virulent PCV2b and PCV2a strains.

### Example 3

This study evaluated the efficacy of Porcine Circovirus Type 2 ORF2b Prototype Vaccine (including recombinant baculovirus expressed PCV2b ORF2 protein) against a PCV2 ORF2b (PCV2b) challenge when given at three weeks of age.

Forty two healthy CDCD pigs (11 pigs from each of 2 litters and 10 pigs from each of 2 litters) were blocked and housed amongst six pens. Pigs within a pen were equally randomized to 1 of 3 treatment groups: Group 1 (Strict Negative Controls) consisted of 6 pigs and received no treatment, Group 2 (Challenge Controls, n=18) received no treatment, and Group 3 (Experimental PCV2b vaccine, n=18). An overview of the treatment groups is provided in Table 1.

**Table 1:**

| **Group** | **No. of Pigs** | **Treatment** | **Day 0** | **Day 11 and Day 17** | **Day 14** | **Day 42** |
|---|---|---|---|---|---|---|
| 1 | 6 | Strict Neg Cont | n/a | n/a | Necropsy | n/a |
| 2 | 18 | none | n/a | KLH/ICFA Treatment | PCV2b challenge | Necropsy |
| 3 | 18 | PCV2b ORF2 protein + Carbopol | Vaccin ate | KLH/ICFA Treatment | PCV2b challenge | Necropsy |

On D0 pigs were 24 days of age and Group 3 pigs were administered a 1 mL dose of vaccine intramuscularly (IM). On D11 and D17, all pigs received a 2.0 mL dose of KLH/ICFA, intramuscularly (IM). One pig was removed from Group 2 before challenge (poor condition) and two pigs were removed from group 3 before challenge (group B *Salmonella* species, *S*. *agona*)*.* On D14 all pigs were challenged with 5.25 log₁₀TCID₅₀/mL of live virulent PCV2b 1.0 mL IM in the right neck and 1.0 mL intranasally. Pigs were examined daily for overall health. Blood samples were collected on D-4, D14, D21, D28, D33 and D42, and sera were tested for PCV2 viremia by quantitative real time polymerase chain reaction on all days with the exception of Day -4. 18 percent (3/17) of the pigs of the control group died before the end of the study in comparison with 0 percent of the pigs (0/16 pigs) in the vaccinated group. In the control group, two pigs on D39 and one pig on D40 were moribund or found dead, in comparison with none of the pigs in the vaccinated group until the end of the study. On D42 all surviving pigs were euthanized; a tonsil sample, a tracheobronchial lymph node, a mesenteric lymph node and an iliac lymph node were collected into formalin. A pathologist scored each tissue as negative or positive (severity score of 1 to 3) for lymphoid depletion, inflammation and colonization (immunohistochemistry).

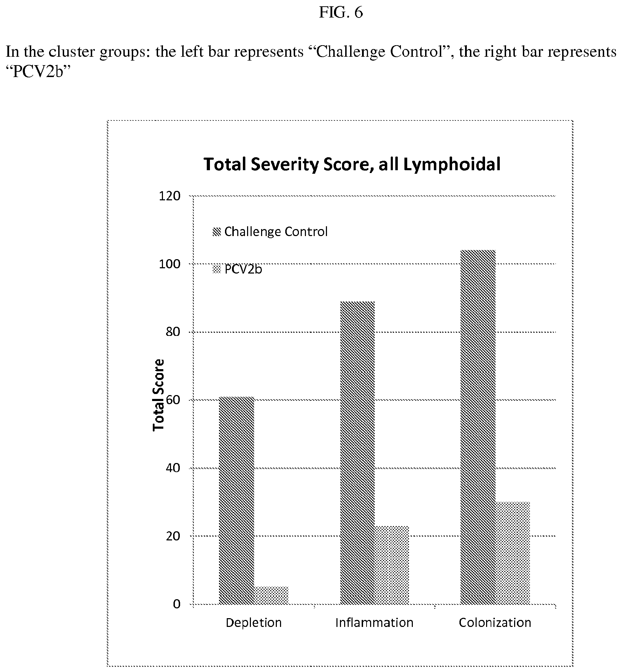

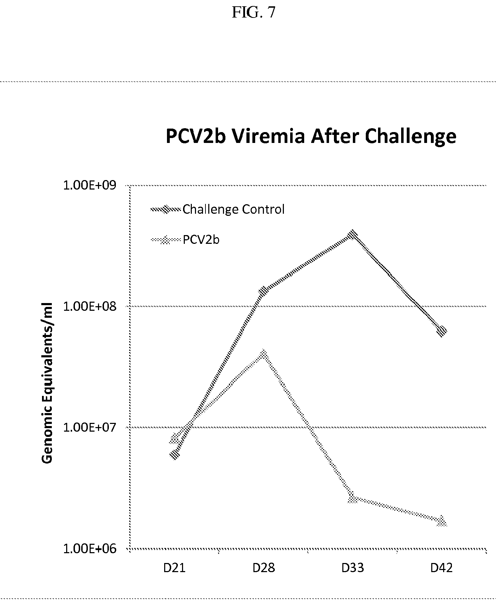

Based on histological results, the mitigated fraction (and lower confidence interval) for lymphoid depletion was 0.511 (0.4043), for lymphoid inflammation was 0.392 (0) and for lymphoid colonization was 0.617 (0.4), **Figure 6**. Thus, it was observed that the mitigation of lymphoid depletion, lymphoid inflammation and lymphoid colonization was significant in the vaccinated pigs, in comparison with the challenge control. Also, analysis of the quantitative rt-PCR viremia data by using PCV2b orf2 specific primers (Opriessnig et al. Vet Res. 2010 41(3):31, which is incorporated herein by reference) revealed a reduction of viral load in vaccinated pigs in comparison with the challenge control over time (**Figure 7**).

Within the context of the invention made and the experimental data provided herewith, in particular the following was considered:
- with respect to lymphoid depletion: to support evidence of "aid in the prevention of lymphoid depletion", a pig was considered positive if one or more of the 4 lymphoid tissue samples (tonsil, TBLN, MLN or ILN) was histologically positive for lymphoid depletion;
- with respect to lymphoid inflammation: to support evidence of "aid in the prevention of lymphoid inflammation", a pig was considered positive if one or more of the 4 lymphoid tissue samples (tonsil, TBLN, MLN or ILN) was histologically positive for lymphoid inflammation;
- with respect to lymphoid colonization: to support evidence that pigs cleared infection by 4 weeks post-virus exposure, a pig was considered positive if one or more of the 4 lymphoid tissue samples (tonsil, TBLN, MLN or ILN) was positive for PCV2 lymphoid colonization by IHC;
- with respect to viremia: to support evidence of "aid in the prevention of viremia", a pig was considered positive on the day of sampling if the serum rt-PCR test was ≥1.0 x 104 PCV2 genomic equivalents (the linear lower level); and
- with respect to mortality: to support evidence of "aid in the prevention of mortality", a pig was considered positive for mortality if it succumbed to challenge (died or required euthanasia for humane reasons with attributable clinical signs, gross lesions and/or histological lesions consistent with PCV2).

The present invention furthermore relates to the following items:
1. PCV2 subtype b (PCV2b) ORF2 protein or immunogenic composition comprising PCV2b ORF2 protein for use in a method for the treatment or prevention of an infection with PCV2, the reduction, prevention or treatment of clinical signs caused by an infection with PCV2, or the prevention or treatment of a disease caused by an infection with PCV2.
2. The PCV2b ORF2 protein or immunogenic composition comprising PCV2b ORF2 protein for use according to item 1, wherein said PCV2b ORF2 protein is a recombinant PCV2b ORF2 protein, preferably a recombinant baculovirus expressed PCV2b ORF2 protein.
3. The PCV2b ORF2 protein or immunogenic composition comprising PCV2b ORF2 protein for use according to item 1 or 2, wherein said PCV2b ORF2 protein comprises or consists of a sequence that is at least 94% or preferably at least 95% identical to the sequence of SEQ ID No:1.
4. The PCV2b ORF2 protein or immunogenic composition comprising PCV2b ORF2 protein for use according to any one of items 1 to 3, wherein the infection with PCV2 is an infection with PCV2 subtype b (PCV2b) and/or with PCV2 of a subtype other than subtype 2b.
5. The PCV2b ORF2 protein or immunogenic composition comprising PCV2b ORF2 protein for use according to any one of items 1 to 4, wherein the infection with PCV2 is an infection with PCV2 of a subtype other than subtype 2b.
6. The PCV2b ORF2 protein or immunogenic composition comprising PCV2b ORF2 protein for use according to item 4, wherein the infection with PCV2 is a concurrent infection with (i) PCV2b and (ii) PCV2 of a subtype other than subtype 2b.
7. The PCV2b ORF2 protein or immunogenic composition comprising PCV2b ORF2 protein for use according to any one of items 4 to 6, wherein the infection with PCV2 of a subtype other than subtype 2b is an infection with PCV2 subtype a (PCV2a) and/or PCV2 subtype c (PCV2c).
8. The PCV2b ORF2 protein or immunogenic composition comprising PCV2b ORF2 protein for use according to any one of items 4 to 7, wherein the infection with PCV2 of a subtype other than subtype 2b is an infection with PCV2a.
9. The PCV2b ORF2 protein or immunogenic composition comprising PCV2b ORF2 protein for use according to any one of items 1 to 8, wherein the infection with PCV2 is a concurrent infection with (i) PCV2b and (ii) PCV2a.
10. The PCV2b ORF2 protein or immunogenic composition comprising PCV2b ORF2 protein for use according to any one of items 4 to 9, wherein said infection with PCV2b is an infection with a PCV2 comprising a polypeptide that is at least 94% or preferably at least 95% identical to the sequence of SEQ ID NO:1 or comprising a polynucleotide which comprises a sequence encoding a polypeptide that is at least 94% or preferably at least 95% identical to the sequence of SEQ ID NO:1.
11. The PCV2b ORF2 protein or immunogenic composition comprising PCV2b ORF2 protein for use according to any one of items 7 to 9, wherein said infection with PCV2a is an infection with a PCV2 comprising a polypeptide that is at least 94% or preferably at least 95% identical to the sequence of SEQ ID NO:2 or comprising a polynucleotide which comprises a sequence encoding a polypeptide that is at least 94% or preferably at least 95% identical to the sequence of SEQ ID NO:2.
12. The PCV2b ORF2 protein or immunogenic composition comprising PCV2a ORF2 protein for use according to any one of items 1 to 11, wherein
   - the treatment or prevention of an infection with PCV2 is based on or comprises or consists of the induction of an immune response against said PCV2,
   - said clinical signs are selected from the group consisting of lymphoid depletion, lymphoid inflammation, positive IHC for PCV2 antigen of lymphoid tissue, viremia, nasal shedding, pyrexia, reduced average daily weight gain, lung inflammation, positive IHC for PCV2 antigen of lung tissue, increased mortality, or
   - said disease is PMWS.
13. The PCV2b ORF2 protein or immunogenic composition comprising PCV2a ORF2 protein for use according to any one of items 4 to 12, wherein
   - the treatment or prevention of an infection with PCV2 of a subtype other than 2b is based on or comprises or consists of the induction of an immune response against said PCV2 of a subtype other than 2b or the concurrent induction of an immune response against said PCV2 of a subtype other than 2b and PCV2b.
14. PCV2 subtype b (PCV2b) ORF2 protein or immunogenic composition comprising PCV2b ORF2 protein, wherein said PCV2b ORF2 protein is a recombinant PCV2b ORP2 protein, preferably a recombinant baculovirus expressed PCV2b ORF2 protein.
15. The PCV2b ORF2 protein or immunogenic composition comprising PCV2b ORF2 protein according to item 14, wherein said PCV2b ORF2 protein comprises or consists of a sequence that is at least 94% or preferably at least 95% identical to the sequence of SEQ ID No:1.
16. A method for the treatment or prevention of an infection with PCV2, the reduction, prevention or treatment of clinical signs caused by an infection with PCV2, or the prevention or treatment of a disease caused by an infection with PCV2, comprising administering PCV2b ORF2 protein or an immunogenic composition comprising PCV2b ORF2 protein to an animal, wherein said infection with PCV2 is preferably the infection with PCV2 according to any one of items 3-12 and/or wherein said PCV2b ORF2 protein or said immunogenic composition is preferably the PCV2b ORF2 protein or the immunogenic composition of item 14 or 15.
17. Use of PCV2b ORF2 protein or of an immunogenic composition comprising PCV2b ORF2 protein for the preparation of a medicament for the treatment or prevention of an infection with PCV2, the reduction, prevention or treatment of clinical signs caused by an infection with PCV2 or for the treatment or prevention of a disease caused by an infection with PCV2, wherein said infection with PCV2 is preferably the infection with PCV2 according to any one of items 3-12 of and/or wherein said PCV2b ORF2 protein or said immunogenic composition is preferably the PCV2b ORF2 protein or the immunogenic composition of item 14 or 15.
18. The method of item 16 or the use of item 17, wherein
   - the treatment or prevention of an infection with PCV2 is based on or comprises or consists of the induction of an immune response against said PCV2, or wherein the prevention, reduction or treatment of an infection with PCV2 of a subtype other than subtype 2a is based on or comprises or consists of the induction of an immune response against said PCV2 of a subtype other than subtype 2a or the concurrent induction of an immune response against said PCV2 of a subtype other than subtype a and PCV2a,
   - said clinical signs are selected from the group consisting of lymphoid depletion, lymphoid inflammation, positive IHC for PCV2 antigen of lymphoid tissue, viremia, nasal shedding, pyrexia, reduced average daily weight gain, lung inflammation positive IHC for PCV2 antigen of lung tissue, increased mortality, or
   - said disease is PMWS.
19. The PCV2b ORF2 protein or immunogenic composition or the method or the use according to any one the preceding items, wherein said PCV2b ORF2 protein or said immunogenic composition is administered only once.
20. The PCV2b ORF2 protein or immunogenic composition or the method or the use according to any one the preceding items, wherein said PCV2b ORF2 protein or said immunogenic composition is administered to an animal, preferably to a swine, more preferably to a pig, in particular preferably to a piglet or a sow.

## Claims

1. Recombinant baculovirus expressed PCV2 subtype b (PCV2b) ORF2 protein for use in a method for the prevention of an infection with PCV2, the reduction or prevention of clinical signs caused by an infection with PCV2, or the prevention of a disease caused by an infection with PCV2, wherein said PCV2b ORF2 protein comprises or consists of a sequence that is at least 95% identical to the sequence of SEQ ID No: 1, wherein the infection with PCV2 is an infection with PCV2a.

2. The PCV2b ORF2 protein for use according to claim 1, wherein said prevention of an infection with PCV2, reduction or prevention of clinical signs caused by an infection with PCV2, or prevention of a disease caused by an infection with PCV2 is achieved by a single administration of only one dose.

3. The PCV2b ORF2 protein for use according to any one of claims 1 to 2, wherein said infection with PCV2a is an infection with a PCV2 comprising a polypeptide that is at least 94% or preferably at least 95% identical to the sequence of SEQ ID NO:2 or comprising a polynucleotide which comprises a sequence encoding a polypeptide that is at least 94% or preferably at least 95% identical to the sequence of SEQ ID NO:2.

4. The PCV2b ORF2 protein for use according to any one of claims 1 to 3, wherein
- the prevention of an infection with PCV2 is based on or comprises or consists of the induction of an immune response against said PCV2,
- said clinical signs are selected from the group consisting of lymphoid depletion, lymphoid inflammation, positive IHC for PCV2 antigen of lymphoid tissue, viremia, nasal shedding, pyrexia, reduced average daily weight gain, lung inflammation, positive IHC for PCV2 antigen of lung tissue, increased mortality, or
- said disease is PMWS.

5. The PCV2b ORF2 protein for use according to any one of the preceding claims, wherein said PCV2b ORF2 protein is administered to an animal, preferably to a swine, more preferably to a pig, in particular preferably to a piglet or a sow.

6. The PCV2b ORF2 protein for use according to any of the preceding claims, wherein said protein is comprised in an immunogenic composition further comprising an adjuvant, and physiological saline.

7. The PCV2b ORF2 protein for use according to claim 6, wherein said adjuvant is Carbopol.
